# EUROPEAN PATENT SPECIFICATION

(11) **EP 3 124 019 B1**
(45) Date of publication and mention of the grant of the patent: **18.10.2017**
(21) Application number: 15306251.8
(22) Date of filing: 31.07.2015
(51) Int. Cl.: A61K 31/165, A61P 9/02, A61K 31/137

(54) **SUBSTANCE SELECTED AMONG MIDODRINE, A PHARMACEUTICAL SALT AND THE ACTIVE METABOLITE THEREOF, FOR USE IN THE TREATMENT OF OBSTRUCTIVE CARDIOPATHY**
SUBSTANZ, DIE AUSGEWÄHLT IST AUS MIDODRIN, EINEM PHARMAZEUTISCHEN SALZ UND SEINEM AKTIVEN METABOLITEN DAVON, ZUR VERWENDUNG BEI DER BEHANDLUNG OBSTRUKTIVER KARDIOPATHIE
SUBSTANCE SÉLECTIONNÉE PARMI LA MIDODRINE, UN SEL PHARMACEUTIQUE ET LE MÉTABOLITE ACTIF DE CELLE-CI, POUR UNE UTILISATION DANS LE TRAITEMENT D'UNE CARDIOPATHIE OBSTRUCTIVE

(43) Date of publication of application: 01.02.2017
(73) Proprietor: Université de Bordeaux, 33000 Bordeaux (FR); Centre Hospitalier Universitaire de Bordeaux, 33404 Talence (FR)
(72) Inventor: LAFITTE, Stéphane, 33600 PESSAC (FR)
(74) Representative: Novagraaf Technologies

(56) References cited:
- JOÃ GBP O L CAVALCANTE ET AL: "Diversity of Mitral Valve Abnormalities in Obstructive Hypertrophic Cardiomyopathy", PROGRESS IN CARDIOVASCULAR DISEASES, vol. 54, no. 6, 2012, pages 517-522, XP028430424, ISSN: 0033-0620, DOI: 10.1016/J.PCAD.2012.03.002 [retrieved on 2012-03-21]
- TOYOZAKI T ET AL: "Response to sympathomimetic agents in hypertrophic cardiomyopathy and in hypertensive cardiac hypertrophy", JOURNAL OF THE AUTONOMIC NERVOUS SYSTEMS, ELSEVIER, AMSTERDAM, NL, vol. 41, no. 3, 1 December 1992 (1992-12-01), pages 239-240, XP024325049, ISSN: 0165-1838, DOI: 10.1016/0165-1838(92)90087-W [retrieved on 1992-12-01]
- TANEJA I ET AL: "Aortic stenosis and autonomic dysfunction: Co-conspirators in syncope", AMERICAN JOURNAL OF MEDICAL SCIENCES, LIPPINCOTT WILLIAMS & WILKINS, USA, vol. 327, no. 5, 1 January 2004 (2004-01-01), pages 281-283, XP009187071, ISSN: 0002-9629
- SCHRAGE WILLIAM G; EISENACH JOHN H; DINENNO FRANK A; ROBERTS SHELLY K; JOHNSON CHRISTOPHER P; SANDRONI PAOLA; LOW PHILIP A; JOYNER: "Effects of midodrine on exercise-induced hypotension and blood pressure recovery in autonomic failure", JOURNAL OF APPLIED PHYSIOLOGY., vol. 97, no. 5, 23 July 2004 (2004-07-23), pages 1978-1984, XP055226681, US ISSN: 8750-7587, DOI: 10.1152/japplphysiol.00547.2004
- WIELING W ET AL: "Reflex syncope in children and adolescents", HEART 200409 GB, vol. 90, no. 9, September 2004 (2004-09), pages 1094-1100, XP002750823, ISSN: 1355-6037
- Barnsley Hospital NHS Foundation Trust: "Guidance for the use of Midodrine in patients with Postural Hypotension", , 1 March 2011 (2011-03-01), pages 1/2-2/2, XP002750824, Retrieved from the Internet: URL:http://www.barnsleyccg.nhs.uk/CCG%20Do wnloads/Members/Medicines%20management/Pre scribing%20Guidelines/Midodrine%20prescrib ing%20information.pdf [retrieved on 2015-11-10]

## Description

### Technical field

The present invention refers to a substance for use in the treatment of obstructive cardiopathy.

Therefore, the present invention has utility in the medical and pharmaceutical fields.

In the description below, the references into brackets **([ ])** refer to the listing of references situated at the end of the text.

### Background of the Invention

Hypertrophic cardiomyopathy (HCM) is the most common heritable cardiovascular disorder, affecting 0.2% to 0.5% of the general population, and is a leading cause of sudden cardiac death in young athletes (Maron BJ.: "Contemporary insights and strategies for risk stratification and prevention of sudden death in hypertrophic cardiomyopathy", Circulation 2010; 121:445-56 **[1]**). The occurrence of hypertrophic cardiomyopathy is a significant cause of sudden unexpected cardiac death in any age group and as a cause of disabling cardiac symptoms. Younger people are likely to have a more severe form of hypertrophic cardiomyopathy.

HCM is frequently asymptomatic until sudden cardiac death, and for this reason some suggest routinely screening certain populations for this disease.

With HCM, the myocytes in the heart increase in size, which results in the thickening of the heart muscle. This increased thickness of heart muscle typically consists of asymmetric septal hypertrophy, and systolic anterior motion (SAM) of the mitral valve. In addition, the normal alignment of muscle cells is disrupted, a phenomenon known as myocardial disarray. HCM also causes disruptions of the electrical functions of the heart.

HCM is most commonly due to a mutation in one of 9 sarcomeric genes that results in a mutated protein in the sarcomere, the primary component of the myocyte (the muscle cell of the heart). These are predominantly single-point missense mutations in the genes for beta-myosin heavy chain (MHC), myosin-binding protein C, cardiac troponin T, or tropomyosin. These mutations cause myofibril and myocyte structural abnormalities and possible deficiencies in force generation.

The clinical course of HCM is variable. Many patients are asymptomatic or mildly symptomatic. The symptoms of HCM include dyspnea (shortness of breath) due to stiffening and decreased blood filling of the ventricles, exertional chest pain (sometimes known as angina) due to reduced or restricted blood flow to the coronary arteries, uncomfortable awareness of the heart beat (palpitations) due to the aforementioned ischemia, as well as disruption of the electrical system running through the abnormal heart muscle, lightheadedness, fatigue, fainting (called syncope) and sudden cardiac death. As mentioned, dyspnea is largely due to increased stiffness of the left ventricle, which impairs filling of the ventricles, but also leads to elevated pressure in the left ventricle and left atrium, causing back pressure and interstitial congestion in the lungs. Symptoms are not closely related to the presence or severity of an outflow tract obstruction. Often, symptoms mimic those of congestive heart failure (especially activity intolerance and dyspnea), but treatment of each is different.

Different treatments exist to improve symptoms of HCM. The primary goal of medications is to relieve symptoms such as chest pain, shortness of breath, and palpitations.

Beta blockers are considered as first-line agents, as they can slow down the heart rate.

For patients who cannot tolerate beta blockers or do not have good control of symptoms with beta blockers, nondihydropyridine calcium channel blockers such as verapamil can be used. These medications also decrease the heart rate, though their use in patients with severe outflow obstruction, elevated pulmonary artery wedge pressure and low blood pressures should be done with caution.

For patients who continue to have symptoms despite the above treatments, disopyramide can be considered for further symptom relief. Diuretics can be considered for patients with evidence of fluid overload, though cautiously used in those with evidence of obstruction. Patients who continue to have symptoms despite drug therapy can consider more invasive therapies.

In this purpose, surgical septal myectomy is an open heart operation done to relieve symptoms in patients who remain severely symptomatic despite medical therapy. It has been performed for more than 25 years. Surgical septal myectomy uniformly decreases left ventricular outflow tract obstruction and improves symptoms, and in experienced centers has a surgical mortality of less than 1%, as well as 85% success rate. However, complications of septal myectomy surgery include possible death, arrhythmias, infection, incessant bleeding, septal perforation/defect, stroke.

Another way of treatment is alcohol septal ablation, which is a percutaneous technique involving injection of alcohol into one or more septal branches of the left anterior descending artery. This is a technique with results similar to the surgical septal myectomy procedure but is less invasive, since it does not involve general anaesthesia and opening of the chest wall and pericardium (which are done in a septal myomectomy). In a select population with symptoms secondary to a high outflow tract gradient, alcohol septal ablation can reduce the symptoms of HCM. In addition, older individuals and those with other medical problems, for whom surgical myectomy would pose increased procedural risk, would likely benefit from the lesser invasive septal ablation procedure. When performed properly, an alcohol septal ablation induces a controlled heart attack, in which the portion of the interventricular septum that involves the left ventricular outflow tract is infarcted and will contract into a scar. However, which patients are best served by surgical myectomy, alcohol septal ablation, or medical therapy is an important topic and one which is intensely debated in medical scientific circles.

Another alternative treatment is the use of a pacemaker that has been advocated in a subset of individuals, in order to cause asynchronous contraction of the left ventricle. Since the pacemaker activates the interventricular septum before the left ventricular free wall, the gradient across the left ventricular outflow tract may decrease. This form of treatment has been shown to provide less relief of symptoms and less of a reduction in the left ventricular outflow tract gradient when compared to surgical myectomy. Technological advancements have also led to the development of a dual-chamber pacemaker, which is only turned on when needed (in contrast to a regular pacemaker which provides a constant stimulus). Although the dual-chamber pacemaker has shown to decrease ventricular outflow tract obstruction, experimental trials have only found few individuals with improved symptoms. Unfortunately, researchers suspect that these reports of "improved" symptoms are due to a placebo effect.

In cases that are refractory to all other forms of treatment, cardiac transplantation is one option. It is also the only treatment available for end-stage heart failure. However, transplantation must occur before the onset of symptoms such as pulmonary vessel hypertension, kidney malfunction, and thromboembolism in order for it to be successful. Studies have indicated a seven-year survival rate of 94% in patients after transplantation.

In two-third of CMH patients, CMH causes an obstruction to blood ejection, whether at rest or during effort. For those patients, resting left ventricular outflow tract obstruction (LVOTO) due to SAM is observed in 25% to 30% and, when severe CMH, may cause dyspnea, chest pain, syncope, and a predisposition to developing atrial arrhythmias (Wigle ED, Sasson Z, Henderson MA, et al. : "Hypertrophic cardiomyopathy. The importance of the site and the extent of hypertrophy. A review", Prog Cardiovasc Dis 1985;28:1-83 **[2]**). For these patients, all the common efforts of routine life are rendered unbearable.

Insights into the pathophysiology of LVOTO have recently been provided by exercise echocardiography, which can quantify the gradient during or after exercise (Maron MS, Olivotto I, Zenovich AG, et al. Hypertrophic cardiomyopathy is predominantly a disease of left ventricular outflow tract obstruction. Circulation 2006;114:2232-9 **[3]**; Shah JS, Esteban MT, Thaman R, et al. Prevalence of exercise-induced left ventricular outflow tract obstruction in symptomatic patients with non-obstructive hypertrophic cardiomyopathy. Heart 2008;94:1288-94 **[4]**; Joshi S, Patel UK, Yao SS, et al. Standing and exercise Doppler echocardiography in obstructive hypertrophic cardiomyopathy: the range of gradients with upright activity. J Am Soc Echocardiogr 2010; 24:75-82 **[5]**; Argulian E, Chaudhry FA. Stress testing in patients with hypertrophic cardiomyopathy. Prog Cardiovasc Dis 2012;54:477-82 **[6]**). Although the Venturi effect was believed to be responsible for SAM ([4]; Maron BJ, Gottdiener JS, Roberts WC, Henry WL, Savage DD, Epstein SE. Left ventricular outflow tract obstruction due to systolic anterior motion of the anterior mitral leaflet in patients with concentric left ventricular hypertrophy. Circulation 1978;57:527-33 **[7]**; Maron BJ, Harding AM, Spirito P, Roberts WC, Waller BF. Systolic anterior motion of the posterior mitral leaflet: a previously unrecognized cause of dynamic subaortic obstruction in patients with hypertrophic cardiomyopathy. Circulation 1983;68:282-93 **[8]**), the most recent evidence for LV obstruction in HCM patients favors the flow drag mechanism causing the mitral valve to be pushed against the septum (Gersh BJ, Maron BJ, Bonow RO, et al. 2011 ACCF/AHA guideline for the diagnosis and treatment of hypertrophic cardiomyopathy: executive summary. A report of the American College of Cardiology Foundation/American Heart Association Task Force on Practice Guidelines. J Am Coll Cardiol 2011;58:2703-38 **[9]**). The mechanism of obstruction is probably also related to other alterations produced by HCM. Experimental and observational data suggest that anterior displacement of the papillary muscles and submitral apparatus is necessary to create sufficient leaflet slack to allow for anterior motion of the mitral leaflet (Cavalcante JL, Barboza JS, Lever HM. Diversity of mitral valve abnormalities in obstructive hypertrophic cardiomyopathy. Prog Cardiovasc Dis 2012;54:517-22 **[10]**; Levine RA, Vlahakes GJ, Lefebvre X, et al. Papillary muscle displacement causes systolic anterior motion of the mitral valve. Experimental validation and insights into the mechanism of subaortic obstruction. Circulation 1995;91:1189-95 **[11]**). Nevertheless, the major potential effects of ventricular loading and myocardial contractility must also be considered. These effects may be exerted both in early systole, for which flow, drag, and pushing force of flow are the dominant hydrodynamic forces for SAM, and at midsystole, for which the displacing force is more prominent (**[5]**). Hence, small variations in preload, afterload, or contractility, such as produced by exertion, may lead to large changes in gradient, usually explaining the amplification of obstruction from rest to exercise or from exercise to recovery.

To date, drug treatments such as beta-blockers or calcium channel blockers are moderately effective and sometimes associated with unacceptable adverse effects in CMH patients with obstruction to blood ejection, whether at rest or during effort. In this respect, dihydropyridine calcium channel blockers are traditionally avoided in patients with evidence of obstruction.

Joao Cavalcante et al. ("Diversity of mitral valve abnormalities in obstructive hypertrophic cardiomyopathy", Progress in cardiovascular diseases, vol. 54, no. 6, 2012, pages 517-522) discloses the physiopathology of obstructive hypertrophic cardiomyopathy and the available surgical options.

Toyozaki et al. ("Response to sympathomimetic agents in hypertrophic cardiomyopathy and in hypertensive cardiac hypertrophy", Journal of the autonomic nervous systems, Elsevier, Amsterdam, vol. 41, no. 3, 1 December 1992, pages 239-240) discloses the use of noradrenaline or isoproterenol for treating hypertrophic cardiomyopathy.

Taneja et al. ("Aorti stenosis and autonomic dysfunction : Co-conspirators in syncope", American Journal of Medical sciences, Lippincott Williams & Wilkins, USA, vol. 327, no. 5, 1 January 2004, 281-283) discloses the physiopathology of aortic stenosis and the available surgical options.

Schrage et al. ("Effects of midodrine on exercise-induced hypotension and blood pressure recovery in autonomic failure", Journal of applied physiology, vol. 97, no. 5, 23 July 2004) discloses the use of midodrine in the treatment of autonomic failure.

Wieling et al. ("Reflex syncope in children and adolescents", Heart 200409 GB, vol. 90, no. 9, September 2004, pages 1094-1100) discloses the use of midodrine in the treatment of syncope.

The document Barnsley Hospital NHS Foundation Trust: "Guidance for the use of midodrine in patients with postural hypotension", 1 March 2011, pages 1/2-2/2 discloses that the use of midodrine is contraindicated in patient affected by hypertrophic obstructive cardiomyopathy.

Unfortunately, this problem of obstruction to blood ejection can also affect other heart conditions, even without associated evident hypertrophy, with the same symptoms as those of CMH.

Thus, a need exists of alternative, more efficient and less toxic treatments of heart diseases with obstruction to blood ejection, in particular CMH with obstruction to blood ejection. The present invention fulfills these and other needs.

### Description of the invention

After important researches on series of obstructive heart diseases, the present Applicants are the first ones to have experimented and observed that midodrine may be very efficient to improve the symptoms of obstruction to blood ejection, while being less toxic on these pathologies than traditional treatments as beta-blockers or calcium channel blockers.

The Applicant surprisingly found that midodrine improves exercise breathlessness, exercise chest pain, exercise discomfort and exercise dizziness in patients with an obstructive heart disease.

In this purpose, the Applicant demonstrated an improvement of the venous return, an immediate decrease of the intraventricular obstruction and a decrease of the hyperkinetic state in patients due to administration of midodrine. Surprisingly, the Applicants demonstrated that the immediate increase of venous return allowed decreasing the intraventricular obstruction. The Applicants also surprisingly observed an inverted phenomenon (relapse and increase of obstruction) after few hours stop of the administration of midodrine, hypothesizing the major role of midodrine in balancing the total blood pool from the veins towards the heart.

Accordingly, a substance selected among midodrine, a pharmaceutical salt, a prodrug or an active metabolite thereof, for use in the treatment of obstructive cardiopathy are described.

In a first aspect, the present invention provides a substance selected among midodrine, desglymidodrine and a pharmaceutical salt thereof, for use in the treatment of obstructive cardiopathy.

"Midodrine" refers herein to an ethanomaline derivative having the following formula (I):

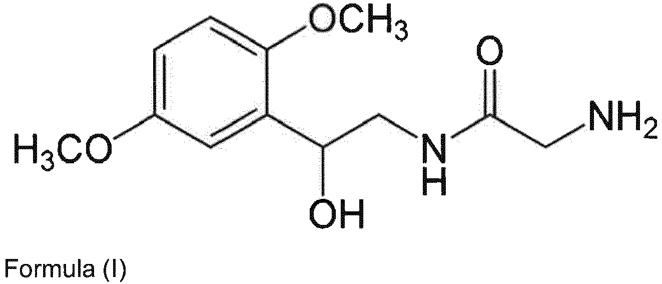

It may be designated under its chemical name 2-amino-N-[2-(2,5-dimethoxyphenyl)-2-hydroxyethyl]acetamide hydrochloride. Its CAS Number is 3092-17-9. Midodrine is a prodrug which forms an active metabolite, desglymidodrine, which has a selective sympathomimetic effect on peripheral alpha-adrenergic receptors and exerts its actions via activation of the alpha-adrenergic receptors of the arteriolar and venous vasculature, producing an increase in vascular tone and elevation of blood pressure. Administration of midodrine results in vasoconstriction of veins at first, thereby reducing the venous pool, and then, in a second time, of arteries. The Applicants hypothesize that this mechanism may be implied in the biological effect of midodrine on the improvement of the symptoms of obstructive cardiopathy observed after administration of midodrine to the patients. Alpha-1-adrenergic receptor agonist substances include midodrine, norepinephrine, dopamine, ephedrine, phenylpropanolamine, methoxamine, phenylephrine, and noradrenaline, or a pharmaceutical salt thereof. Examples of alpha-1-adrenergic receptor agonist substances are disclosed in Goodman and Gilman's the pharmacological basis of therapeutics, eleventh edition, chapter 10, pp 271-295, 2006 (**[12]**).

Midodrine was approved in the United States by the Food and Drug Administration (FDA) in 1996 for the treatment of dysautonomia and orthostatic hypotension.

Dysautonomia (or autonomic dysfunction, autonomic neuropathy) is an umbrella term for various conditions in which the autonomic nervous system (ANS) malfunctions. Dysautonomia is a type of neuropathy affecting the nerves that carry information from the brain and spinal cord to the heart, bladder, intestines, sweat glands, pupils, and blood vessels.

Orthostatic hypotension, also known as postural hypotension, orthostasis, and colloquially as head rush or dizzy spell, is a form of low blood pressure in which a person's blood pressure falls when suddenly standing up or stretching. In medical terms, it is defined as a fall in systolic blood pressure of at least 20 mmHg or diastolic blood pressure of at least 10 mmHg when a person assumes a standing position.

The term "pharmaceutical salt" is meant to include any pharmaceutically acceptable salt, the substance that are prepared with relatively nontoxic acids or bases, depending on the particular substituents found on the compounds described herein. Examples of non toxic pharmaceutically acceptable salts may include hydrochloride, sulfate, pyrosulfate, bisulfate, sulphite, bisulphite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutylate, caprate, heptanoate, propiolate, oxalate, malonate, succinate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutylate, citrate, lactate, hydroxybutylate, glycolate, malate, tartrate, methanesulfonate, propanesulfonate, naphthalene-1-sulfonate, naphthalene-2-sulfonate and mandelate. Pharmaceutically acceptable salt of midodrine may be midodrine hydrochloride, for example Gutron^{™} (Takeda).

"Active metabolite" refers herein to any therapeutically active metabolite released by activation of the prodrug, i.e. the alpha-1-adrenergic receptor agonist substance within the human body by an enzymatic hydrolysis. According to the present invention it refers to desglymidodrine, which is the active metabolite of midodrine. Advantageously, desglymidodrine acts by a stimulation of alpha-1 adrenergic receptors of the arteriolar and venous vasculature, producing an increase in vascular tone and elevation of blood pressure.; it diffuses poorly across the blood-brain barrier, and is therefore not associated with effects on the central nervous system.

« Prodrug » refers herein to any precursor compound which may be administered in a pharmacologically inactive form, and which is likely to be converted in an active form through a normal metabolic process in physiological conditions. The active form is midodrine or the active metabolite of midodrine desglymidodrine.

"Obstructive cardiopathy" refers herein to any heart disease having an intraventricular obstruction. For example, the intraventricular obstruction may occur at rest, i.e. when oxygen consumption of the body is stable, and/or during exercise, i.e. when oxygen consumption increases due to the realization of a movement by the body and/or throughout the recovery phase after exercise, meaning within the next 5 to 10 minutes after the exercise has stopped. Exercise may be for example, a situation when a body assumes a standing position, or walking, or climbing stairs, or lifting a load. The intraventricular obstruction corresponds to the existence of a pressure gradient observable by Doppler echocardiography, especially of left ventricle structure and function, at rest and/or during exercise. The gradient may be above 30 mmHg at rest, for example strictly above 35 mmHg, or above 40 mmHg, or above 50 mmHg, or above 60 mmHg, or above 70 mmHg, or above 100 mmHg, or for example of about 130 mmHg. The gradient may be above 50 mmHg during exercise, for example above 55 mmHg, or above 60 mmHg, or above 80 mmHg, or above 100 mmHg, or above 110 mmHg, or above 150 mmHg, or for example of about 180 mmHg. Resting echocardiography may be a resting 2-dimensional (2D) echocardiography for example performed according to American Society of Echocardiography guidelines (Lang RM, Bierig M, Devereux RB, et al. Recommendations for chamber quantification: a report from the American Society of Echocardiography's Guidelines and Standards Committee and the Chamber Quantification Writing Group. J Am Soc Echocardiogr 2005;18: 1440-63 **[13]** ; Gottdiener JS, Bednarz J, Devereux R, et al. American Society of Echocardiography recommendations for use of echocardiography in clinical trials. J Am Soc Echocardiogr 2004;17:1086-119 **[14]**), with ultrasound recordings by an experienced (level 3) operator (Quinones MA, Douglas PS, Foster E, et al. ACC/AHA clinical competence statement on echocardiography: a report of the American College of Cardiology/American Heart Association/American College of Physicians-American Society of Internal Medicine Task Force on Clinical Competence. J Am Coll Cardiol 2003;41:687-708 **[15]**). Exercise echocardiography may be performed for example as bicycle exertion in a semisuspine position (50°) to enable simultaneous transthoracic echocardiography during exercise.

The obstructive cardiopathy may be an obstructive cardiomyopathy. It may be selected among hypertrophic cardiomyopathy, hypertensive cardiopathy, a primary hyperkinetic function, a malposition of papillary muscles and a situation with primary hyperkinesia of left ventricle in non hypertrophic cardiomyopathy.

"Treatment" refers herein to any improvement of at least one symptom of the obstructive cardiopathy, or to the recovery of the obstructive cardiopathy, or to the prevention of the symptoms of the obstructive cardiopathy. The recovery of the obstructive cardiopathy may be a decrease or a disappearance of the obstruction. The symptoms of the obstructive cardiopathy may be anyone of those of the New York Heart Association (NYHA) Functional Classification. The at least one symptom that may be improved may be selected among breathlessness, especially exercise breathlessness, chest pain, especially exercise chest pain, discomfort, especially exercise and dizziness, especially exercise dizziness. The improvement of at least one symptom may refer to total or partial disappearance of at least one symptom, which may be transient or definitive, at rest and/or during exercise. The improvement of at least one symptom may refer for example to a decrease of NYHA class according to NYHA Functional Classification.

Advantageously, the substance may increase the venous return of blood to heart, thus decreasing the hyperkinetic state of the cardiopathy; i.e. decreasing the heart contraction and/or decreasing heart flow rate, and/or decreasing circulatory rate. Advantageously, the substance may increase loading conditions, i.e. afterload and preload.

Advantageously, the substance may be administered to a patient in need thereof to a pharmaceutically acceptable and efficient dose for the treatment of obstructive disease.

Advantageously, it may be a dose increasing the venous return of blood to heart. Advantageously, the substance is administrated from one to eight times per 24 hours, i.e. per day. For example, the dose(s) may be administrated once per day, or twice per day, or three times per day, or four times per day, or five times per day, or more until eight times per day. For example, it may be administrated from one to three, or to one to four times per day, i.e. per 24 hours. For example, the total dose administrated per day may be comprised of from 6 mg to 45 mg per day, i.e. per 24 hours. For example, the substance may be administered from 1 to 3 dose(s) of 2.5 mg each of the substance, from 1 to 3 times per day, or from 1 to 4 times per day. For example, the dosing regimen may be advantageously selected to maintain the number of drug intakes per day (i.e. per 24 hours) when searching for the more adapted dosing regimen for a patient, while increasing the quantity of administered substance per drug intake. It may correspond to an administration of from about 0.1 mg of the substance / kg of patient, to about 0.75 mg of the substance / kg of patient, per day. In the particular case where the patient is an infant, the man skilled in the art would adapt the dosing regimen according to his general technical knowledge.

According to the invention, the substance may be administered for sufficient time for the treatment of obstructive cardiopathy as defined above. In this purpose, the substance may be administered for one month, or for two months, or for three months, or for six months, or for at least six months, or for the patient's lifetime.

The substance may be in any suitable form for an administration to a human or an animal, especially in the form of a medicament. The animal may be selected from the group comprising felids, for example cat, canids, for example dog, cervids, equids, mustelids, procyonids, viverrids and ursids.

Administration may be carried out directly, i.e. pure or substantially pure, or after mixing of the substance with a pharmaceutically acceptable carrier and/or medium.

Administration of the substance may be carried out either simultaneously, separately or sequentially with at least one another active compound(s) selected in the group comprising beta-blockers, for example propranolol, bisoprolol, carvedilol and nadolol, calcium inhibitors, for example verapamil, antiarythmic agents, for example amiodarone, and anticoagulant agents, for example rivaroxaban. The substance and the at least one another active compound(s) mixture may be administered in a relative amount of 0:1 to 1:0, for example 1:1. The other active compound may be used for the treatment of obstructive cardiopathy, or for another pathology existing together with the obstructive cardiopathy, for example an orthostatic hypotension, or a rhythm disorders such as atrial fibrillation.

In one embodiment, the administration may be an oral administration. In this embodiment, it may be a buccal or a sublingual administration. It may for example be in the form selected from the group comprising a liquid formulation, an oral effervescent dosage form, an oral powder, a pill, a multiparticule system, an orodispersible dosage form, a solution, a syrup, a suspension, an emulsion and oral drops. When the medicament is in the form of an oral effervescent dosage form, it may be in a form selected from the group comprising tablets, granules, powders. When the medicament is the form of an oral powder or a multiparticulate system, it may be in a form selected from the group comprising beads, granules, mini tablets and micro granules. When the medicament is the form of an orodispersible dosage form, it may be in a form selected from the group comprising orodispersible tablets, lyophilized wafers, thin films, a chewable tablet, a tablet and a capsule, a medical chewing gum. According to the present invention, when the medicament is for buccal and sublingual routes, it may be selected from the group comprising buccal or sublingual tablets, muco adhesive preparation, oro-mucosal drops and sprays.

In another embodiment, the administration is a parenteral administration. The parenteral administration may be selected from the group comprising intravenous administration, intramuscular administration and subcutaneous administration. In those cases, the substance may be in the form of an injectable solution.

In another embodiment, the administration may be a transdermal or a transmucosal administration. When the medicament is for topical-transdermal administration, it may be selected from the group comprising ointments, cream, gel, lotion, patch and foam. It may also be a medicament for nasal administration, for example selected from the group comprising nasal drops, nasal spray, nasal powder.

In another embodiment, the administration may be a rectal administration, for example suppository or hard gelatin capsule.

The skilled person in the art understands clearly that the term "form" as used herein refers to the pharmaceutical formulation of the medicament for its practical use. For example, the medicament may be in a form selected from the group comprising an injectable form (for example as Avlocardyl®5mg/ml), syrup (for example as Efferalgan^{®}3%), oral suspension (for example as Efferalgan^{®}3%), a pellet (for example as Dafalgan^{®}1g), powder (for example as Doliprane^{®}100mg), granules (for example as Zoltum^{®}10mg), spray, transdermal patch (for example as Cordipatch^{®}5mg/24h) or local form (cream, lotion, collyrium) (for example as Dermoval creme^{®}, as Betneval^{®}lotion and as Chibroxine^{®} collyre respectively).

In these examples, the substance, for example midodrine, may be added or may replace the active ingredient(s) of said medicaments.

The pharmaceutically acceptable carrier may be any know suitable pharmaceutically carrier used for the administration of a substance to a human or to an animal, depending on the subject. For example, this carrier may be one or more carrier(s) selected from the group comprising for example the monomethoxy-polyethyleneglycol (for example as in Viraferonpeg^{®}), Liposome (for example as in Ambizome^{®}), magnesium stearate (E572), talc (E553b), Silicon dioxide (E551), microcrystalline cellulose (E460) and maize starch. Preferably, the carrier comprises magnesium stearate (E572), talc (E553b), Silicon dioxide (E551), microcrystalline cellulose (E460) and maize starch.

The medium may be any know medium used for the administration of a substance to a human or to an animal. For example, this medium may be selected from the group comprising for example cremophor (for example as in Sandimmun^{®}) or cellulosis (for example as in Avlocardyl^{®} LP160mg).

The pharmaceutical form of the drug is selected with regard to the human or animal to be treated. For example, for a child or a baby, a syrup or an injection is preferred. Administration may be carried out with a weight graduated pipette.

In a second aspect, the present disclosure relates to a method of treating a subject suffering from an obstructive cardiopathy as defined above, comprising a step of administering to said subject a substance as defined above.

In another aspect, the present disclosure relates to the use of a substance as defined above for the manufacture of a medicament for the treatment of an obstructive cardiopathy as defined above.
This invention is further illustrated by the following examples with regard to the annexed drawings.

### Brief description of the figures

- Figure 1: represents a 2D echocardiography of left ventricle revealing a bulging septum of thickness: 12-13 mm with incomplete systolic anterior motion of the mitral valve (SAM) at rest. No significant rest obstruction was observed.
- Figure 2: represents a Doppler record of left ventricle systolic flow revealing a severe obstruction up to 100mmHg after exercise.
- Figure 3: represents a 2D echocardiography of left ventricle during exercise with the appearance of a complete SAM.
- Figure 4: represents a 2D echocardiography at rest in which systolic left ventricular volume in this HCM patients was minimal at least virtual with a physical contact between septal and lateral walls.
- Figure 5: represents a Doppler echocardiography at rest with a significant obstruction above of 80mmHg.
- Figure 6: represents a Doppler echocardiography exercise. The obstruction was increased with a maximum gradient of 118mmHg.
- Figure 7: represents an exercise stress Doppler echocardiography during chest pain experienced in everyday life obtained after a short distance walk (less than 20 meters). An intra ventricular obstruction up to 145 mmHg is recorded.
- Figure 8: represents an exercise stress Doppler echocardiography during chest pain experienced in everyday life obtained after a short distance walk (less than 20 meters). The parietal contact between the septal and lateral walls was increased.
- Figure 9: represents a Doppler echocardiography during a leg-raise test, that reveals an immediate reduction in the intraventricular obstruction, measured at 80mmHg.
- Figure 10: represents an echocardiographic follow-up examination under treatment revealing a significantly-reduced maximum gradient of the left intraventricular obstruction at rest, down to 30mmHg in comparison with 80mmHg recorded before treatment initiation.
- Figure 11: represents an echocardiographic follow-up examination under treatment revealing a significantly-reduced maximum gradient of the left intraventricular obstruction at 60mmHg post-exercise compared to 140mmHg before treatment.
- Figure 12: represents an echocardiographic revealing an hypertrophic cardiomyopathy (HCM), obstructive at rest, with a gradient superior to 100mmHg, along with high-grade mitral insufficiency in a hyperkinetic left ventricle.
- Figure 13: represents a Doppler echocardiography during a leg-raise test to increase venous return under echocardiographic control, which instantly reduced the mitral insufficiency to a mild grade and the obstruction to 40mmHg.
- Figure 14: represents a Doppler echocardiography at rest, showing absence of obstruction (maximum gradient of 18mmHg), a mitral insufficiency of a milder degree, and pulmonary pressures normal (32mmHg).
- Figure 15: represents an immediate post-exercise echocardiography Doppler after walking and stair stress tests (50 meters walking and 30 steps), revealing similar characteristics as those of the resting echocardiography (absence of obstruction with a maximum gradient of 18mmHg).

### Examples

### Example 1: Description of a first clinical case

The first patient was 45 years old and admitted to general cardiology at Bordeaux University Hospital (CHU Bordeaux) for orthostatic hypotension following a previous diagnosis of obstructive cardiomyopathy (confirmed by a MYH7 gene mutation). Treatment with Nadolol at 80 mg/day was unable to prevent several lipothymic and syncopal episodes. He also complained of significant discomfort when walking, classed as New York Heart Association (NYHA) dyspnea 2-3.

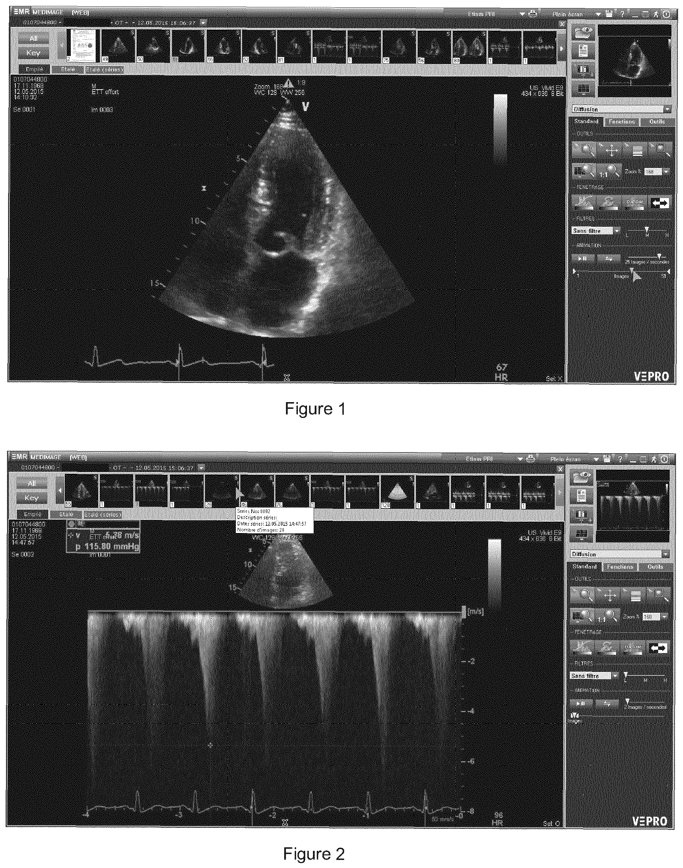

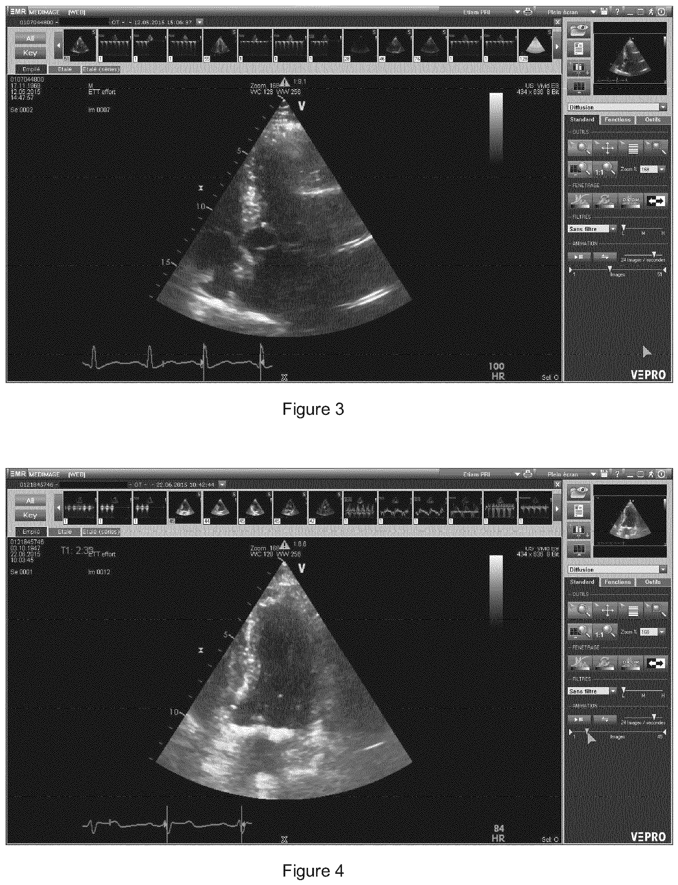

Echocardiography revealed bulging septum (thickness: 12-13 mm) with incomplete systolic anterior motion of the mitral valve (SAM) at rest (Figure 1). There was no intra-ventricular obstruction at rest, yet during exercise it was recorded at 50mmHg gradient, which increased during early recovery to 100mmHg (Figures 2 and 3). Concurrently, the patient presented clinically with his usual symptoms of faintness and dizziness.

Tilt test was performed during his hospitalization, revealing clear orthostatic hypotension after NATISPRAY administration with decreasing blood pressure from 124/80mmHg to 80/48mmHg, also accompanied by faintness, hot flashes, extreme paleness, and blackout.

Monitoring with continuous ECG during hospitalization revealed no heart rhythm or conduction disorders.

Considering the patient's orthostatic hypotension, a treatment with midodrine hydrochloride (Gutron™) was initiated at a dose of 3 2.5-mg tablets per day. This drug produces a direct and selective sympathomimetic effect on peripheral alpha-adrenergic receptors, resulting in vasoconstriction of first the veins (reducing the venous pool) then the arteries. This prevents orthostatic disorders, increases peripheral resistance, and causes a rise in blood pressure.

After 1 month of treatment, faintness did not recur. In addition, the patient reported less shortness of breath during exercise (NYHA severe 2 to severe 1).

Following 6 weeks of treatment, the patient exhibited no longer symptoms or limitations during exercise. Echocardiography demonstrated a total disappearance of obstruction at rest, during exercise or recovery.

We therefore conclude that midodrine hydrochloride, initially prescribed for orthostatic hypotension in this patient with intra-ventricular obstruction, also resolved shortness of breath during exercise. We believe this to be the result of an improvement in cardiac filling, hence resulting in left ventricular cavity dilatation and reduction of the intra-ventricular obstruction responsible for the symptoms.

### Example 2: Description of a second clinical case

The second patient, of female gender, suffered from familial hypertrophic cardiomyopathy (HCM) and was admitted for disabling chest pain during exercise accompanied by shortness of breath during even the mildest activity (walking for less than 10m).

The complete work-up of her cardiomyopathy performed 6 months earlier had revealed:
- Echocardiography: septal obstructive predominant hypertrophic cardiomyopathy (HCM) at 14 mm thickness in the septum, subaortic obstruction with a maximum gradient of 88mmHg, SAM, Grade 2 mitral insufficiency, preserved left ventricular ejection fraction, and absence of aortic valvulopathy.
- Exercise stress echo test: exercise stress ultrasound with no medication set at 92% of the theoretical maximum heart rate and being clinically, electrically, and echographycally negative for ischemia. Obstruction at rest, resolving during exercise and increasing during recovery (90mmHg), absence of arrhythmia, appropriate blood pressure profile.
- Cardiac magnetic resonance imaging (MRI): obstructive HCM with slight SAM, and preserved segmental and global left ventricular function.

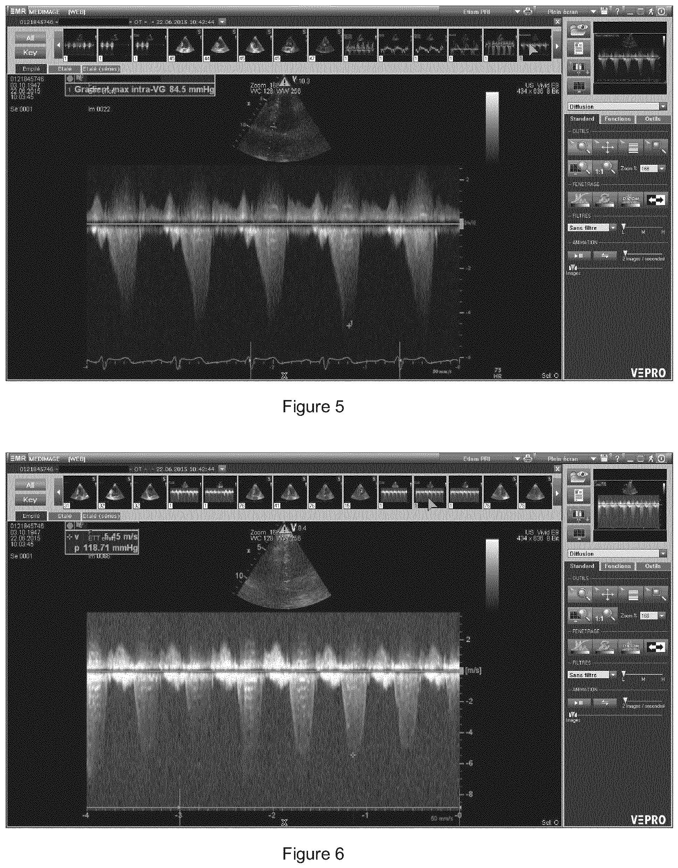

During her hospital stay this time, she underwent an exercise stress echocardiography. Resting echocardiography revealed an obstructive HCM with a gradient of 80mmHg (Figures 4 and 5), along with Grade 2 mitral insufficiency with no increase in filling pressures, and her pulmonary pressures were estimated at 35mmHg.

During exercise, performed on a bicycle at 75 watts and 90% of theoretical maximum heart rate, her blood pressure adapted well and no electrical modification or degradation of the left ventricular function was observed. The obstruction was seen to increase, with a maximum gradient of 120mmHg (Figure 6), yet the patient exhibited few symptoms, presenting solely with a Grade 1-2 dyspnea with no reproduction of pain.

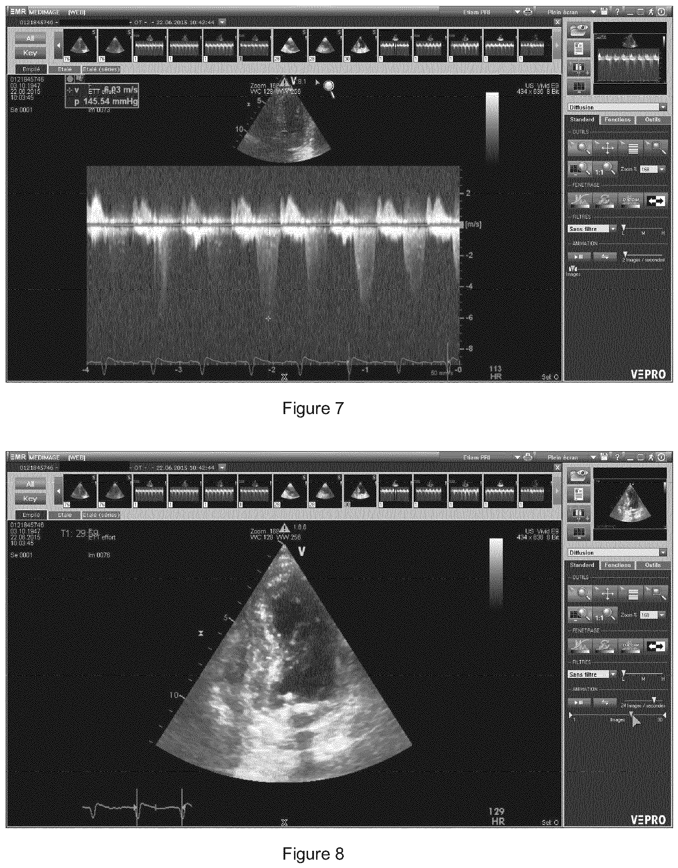

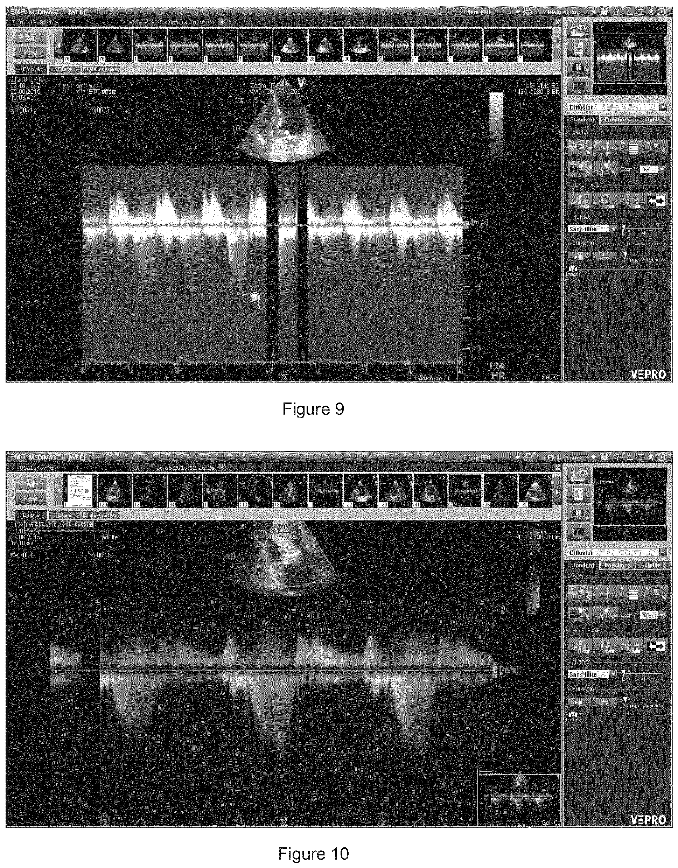

It was also decided to attempt to reproduce the chest pain experienced in everyday life, i.e., when walking, which caused debilitating and violent pain, with a maximum gradient of 145mmHg (Figure 7) and featuring significant parietal contact between the septal and lateral walls (columns) (Figure 8). On performing a leg-raise test, echocardiography revealed an immediate reduction in the intraventricular obstruction, measured at 80mmHg (Figure 9). ECG tracings recorded throughout this test revealed no change indicative of myocardial ischemia.

Coronary angiography was performed to investigate suspicious pain but revealed no anomalies.

Given the leg-raise test results, a treatment aimed at increasing cardiac filling was considered. The test was also repeated with compression stockings, which had no beneficial effect on the patient's comfort.

Considering the improvement achieved by administering Gutron^{™} to the first patient and the debilitating nature of this patient's chest pain, a treatment attempt was made with Gutron^{™}, administered initially in the form of 2.5mg tablets. The patient received first one 2.5mg tablet three times per day and then two 2.5mg tablets three times per day. An increase of the effect has been observed. Within 48 hours of initiating treatment, the patient became asymptomatic, relieved of chest pain, and exhibited no more shortness of breath during exercise (tested by means of two flights of stairs ascension while being monitored medically, with no discomfort experienced).

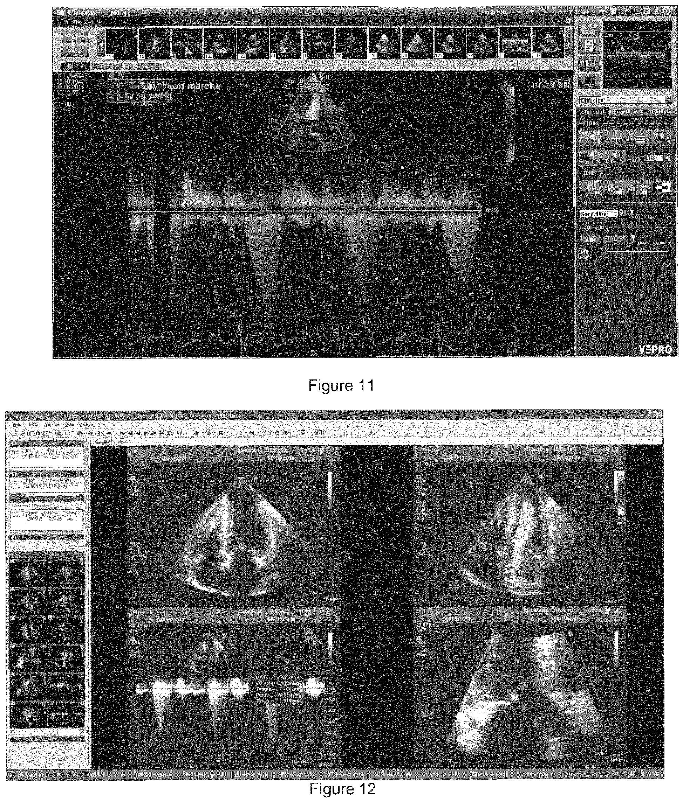

Echocardiographic follow-up examination under treatment revealed a significantly-reduced maximum gradient of the left intraventricular obstruction at rest, down to 30mmHg in comparison with 80mmHg recorded before treatment initiation (Figure 10), and 60mmHg post-exercise compared to 140mmHg before treatment (Figure 11). It is believed that the left ventricular filling was improved by this vasoactive treatment, which also caused a decrease in the intraventricular obstruction.

The patient was discharged. Two weeks following her departure, a follow-up interview was conducted by telephone, in which the patient reported continued improvement with near-absence of chest pain, which no longer occurred every day, nor limited her daily activities. She evaluated her quality of life to have improved from 2/10 to 7/10, and reported the complete absence of shortness of breath when she exercises.
The patient was then asked to stop the midodrine hydrochloride (Gutron^{™}) for 2 days. After only 4 hours, she starting feeling limited during light exertion with reappearance of chest pains and shortness of breath. Immediate re-absorption of Gutron^{™} released the symptoms.

### Example 3: Description of a third clinical case

This 66-year-old patient had been diagnosed many years previously with hypertrophic cardiomyopathy (HCM) and was fitted with a dual-chamber pace maker due to a paroxysmal atrioventricular block. He also presented an early-stage chronic obstructive bronchopneumopathy, caused by smoking, limiting the possibility of beta-blocker prescription. His medical history notably included the sudden death of a sister at 35 years old, potentially indicating a family history of HCM.

The patient complained of dyspnea in everyday activities, classed as NYHA severe 2 (walking 10 meters and stopping due to shortness of breath). He was treated with 2.5mg carvedilol, 200mg amiodarone, and 20mg rivaroxaban.

He was admitted to the emergency room for acute respiratory distress secondary to an acute pulmonary edema. Echocardiography revealed a HCM, obstructive at rest, with a gradient superior to 100mmHg, along with high-grade mitral insufficiency in a hyperkinetic left ventricle (Figure 12). With the appropriate medical treatment, he was able to recover a stable hemodynamic status within 3 days, and his cardiac insufficiency symptoms were reduced. His pace maker was also updated, and a coronary angiography was performed, coming back normal.

However, 1 week later, a further echocardiography was performed that revealed functional characteristics very similar to those observed on the first echocardiography, with a still-present obstruction at 90mmHg, high-grade mitral insufficiency, and pulmonary pressures of approximately 55mmHg, indicating a fragile hemodynamic status.

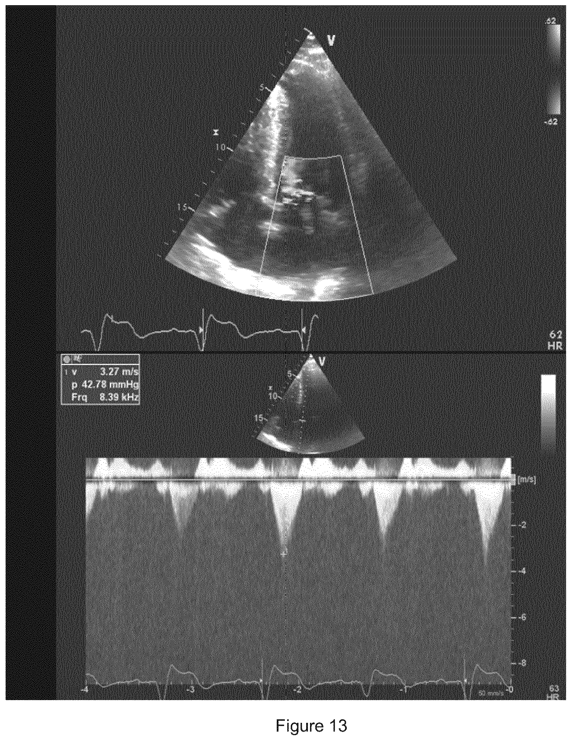

It was then decided to perform the leg-raise test to increase venous return under echocardiographic control, which instantly reduced the mitral insufficiency to a mild grade and the obstruction to 40mmHg (Figure 13). Given the absence of immediate treatment options, the patient was started on a course of midodrine hydrochloride (Gutron^{™}) at a dose of 8 tablets of 2.5mg per day, due to his low blood pressure.

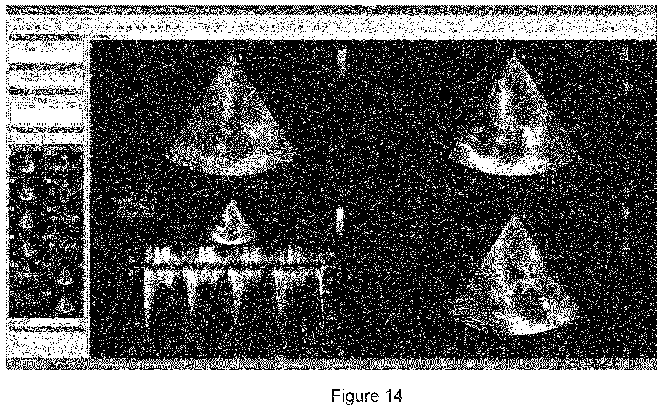

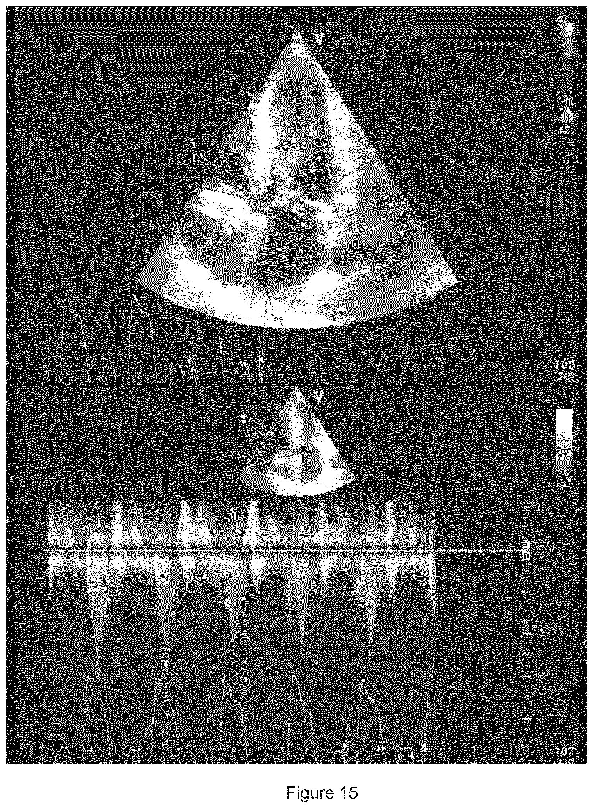

Following 24 hours of treatment, the patient was again evaluated by echocardiography. At rest, the obstruction was entirely absent (maximum gradient of 18mmHg), the mitral insufficiency of a milder degree, and pulmonary pressures normal (32mmHg) (Figure 14). The patient was asked to perform walking and stair stress tests (50 meters walking and 30 steps), which posed him no problem whatsoever, and he experienced no shortness of breath. The immediate post-exercise echocardiography (heart rate: 100 bpm) revealed similar characteristics as those of the resting echocardiography (Figure 15).

The patient was followed up 1 week following treatment initiation, at which point he was still asymptomatic. Echocardiography confirmed his improved hemodynamic profile, with a total absence of the gradient and mitral insufficiency.

### Example 4: Clinical Study

A prospective monocentric, randomized, placebo controlled in parallel, double-blind clinical trial is conducted at the University Hospital Center of Bordeaux. Forty patients with obstruction to blood ejection, whether at rest or during effort, are randomized as follows:
- Arm 1 (20 patients): Midodrine, 5 mg, 3 times a day, 30 days
- Arm 2 (20 patients): Placebo, 5 mg, 3 times a day, 30 days

The main objective of the study is to assess the efficacy of 15 and 30 days midodrine treatment taken as described above on improving the distance covered during a 6 minutes walk test (6MWT).

The six-minute walk test (6MWT) measures the distance an individual is able to walk over a total of six minutes on a hard, flat surface. The goal is for the individual to walk as far as possible in six minutes. A comparison between Arm 1 and Arm 2 is done.

Moreover, the symptoms of HCM including dyspnea, exertional chest pain, uncomfortable awareness of the heart beat are assessed.

Exercise echocardiography is performed before, at 15 and 30 days of treatments in order to measure the impact of treatment upon left ventricular obstruction.

### Reference List

**1.** Maron BJ.: "Contemporary insights and strategies for risk stratification and prevention of sudden death in hypertrophic cardiomyopathy", Circulation 2010;121:445-56.
**2.** Wigle ED, Sasson Z, Henderson MA, et al. : "Hypertrophic cardiomyopathy. The importance of the site and the extent of hypertrophy. A review", Prog Cardiovasc Dis 1985;28:1-83.
**3.** Maron MS, Olivotto I, Zenovich AG, et al. Hypertrophic cardiomyopathy is predominantly a disease of left ventricular outflow tract obstruction. Circulation 2006;114:2232-9.
**4.** Shah JS, Esteban MT, Thaman R, et al. Prevalence of exercise-induced left ventricular outflow tract obstruction in symptomatic patients with non-obstructive hypertrophic cardiomyopathy. Heart 2008;94:1288-94.
**5.** Joshi S, Patel UK, Yao SS, et al. Standing and exercise Doppler echocardiography in obstructive hypertrophic cardiomyopathy: the range of gradients with upright activity. J Am Soc Echocardiogr 2010; 24:75-82.
**6.** Argulian E, Chaudhry FA. Stress testing in patients with hypertrophic cardiomyopathy. Prog Cardiovasc Dis 2012;54:477-82.
**7.** Maron BJ, Gottdiener JS, Roberts WC, Henry WL, Savage DD, Epstein SE. Left ventricular outflow tract obstruction due to systolic anterior motion of the anterior mitral leaflet in patients with concentric left ventricular hypertrophy. Circulation 1978;57:527-33.
**8.** Maron BJ, Harding AM, Spirito P, Roberts WC, Waller BF. Systolic anterior motion of the posterior mitral leaflet: a previously unrecognized cause of dynamic subaortic obstruction in patients with hypertrophic cardiomyopathy. Circulation 1983;68:282-93.
**9.** Gersh BJ, Maron BJ, Bonow RO, et al. 2011 ACCF/AHA guideline for the diagnosis and treatment of hypertrophic cardiomyopathy: executive summary. A report of the American College of Cardiology Foundation/American Heart Association Task Force on Practice Guidelines. J Am Coll Cardiol 2011;58:2703-38.
**10.** Cavalcante JL, Barboza JS, Lever HM. Diversity of mitral valve abnormalities in obstructive hypertrophic cardiomyopathy. Prog Cardiovasc Dis 2012;54:517-22.
**11.** Levine RA, Vlahakes GJ, Lefebvre X, et al. Papillary muscle displacement causes systolic anterior motion of the mitral valve. Experimental validation and insights into the mechanism of subaortic obstruction. Circulation 1995;91:1189-95.
**12.** Goodman and Gilman's the pharmacological basis of therapeutics, eleventh edition, chapter 10, pp 271-295, 2006.
**13.** Lang RM, Bierig M, Devereux RB, et al. Recommendations for chamber quantification: a report from the American Society of Echocardiography's Guidelines and Standards Committee and the Chamber Quantification Writing Group. J Am Soc Echocardiogr 2005;18: 1440-63.
**14.** Gottdiener JS, Bednarz J, Devereux R, et al. American Society of Echocardiography recommendations for use of echocardiography in clinical trials. J Am Soc Echocardiogr 2004;17:1086-119.
**15.** Quinones MA, Douglas PS, Foster E, et al. ACC/AHA clinical competence statement on echocardiography: a report of the American College of Cardiology/American Heart Association/American College of Physicians-American Society of Internal Medicine Task Force on Clinical Competence. J Am Coll Cardiol 2003;41:687-708.

## Claims

1. Substance selected among midodrine, desglymidodrine and a pharmaceutical salt thereof, for use in the treatment of obstructive cardiopathy.

2. Substance for use according to claim 1, wherein said pharmaceutical salt is hydrochloride.

3. Substance for use according to anyone of claims 1 or 2, wherein said obstructive cardiopathy is selected among hypertrophic cardiomyopathy, hypertensive cardiopathy, a malposition of papillary muscles, and a situation with primary hyperkinesia of left ventricle in non hypertrophic cardiomyopathy.

4. Substance for use according to anyone of the preceding claims, wherein said treatment comprises an administration of a dose of said substance comprised of from 6 mg to 45 mg per day.

5. Substance for use according to anyone of the preceding claims, wherein said treatment comprises an administration of said substance which is realized from one to eight times per 24 hours.

6. Substance for use according to anyone of the preceding claims, wherein said treatment includes the improvement of at least one symptom of the obstructive cardiopathy selected among exercise breathlessness, exercise chest pain, exercise discomfort and exercise dizziness.

7. Substance for use according to anyone of the preceding claims, wherein said treatment comprises an oral administration of said substance.

8. Substance for use according to claim 7, wherein said oral administration is a buccal or a sublingual administration.

9. Substance for use according to claim 7, wherein the substance is in a form selected from the group comprising a liquid formulation, an oral effervescent dosage form, an oral powder, a pill, a multiparticule system, an orodispersible dosage form, a solution, a syrup, a suspension, an emulsion and oral drops.

10. Substance for use according to anyone of claims 1 to 6, wherein said treatment comprises a parenteral administration of said substance.

11. Substance for use according to claim 10, wherein said parenteral administration is selected from the group comprising intravenous administration, intramuscular administration and subcutaneous administration.

12. Substance for use according to claim 11, wherein the substance is in the form of an injectable solution.

13. Substance for use according to anyone of the preceding claim, for an administration to a human or an animal selected among from the group comprising felids, canids, cervids, equids, mustelids, procyonids, viverrids and ursids.

14. Substance for use according to anyone of the preceding claims, together with at least one another active compound selected among the group comprising beta-blockers, calcium channel blockers, antiarrhythmic agents, and anticoagulant agents.

## Patentansprüche

1. Stoff, ausgewählt aus Midodrin, Desglymidodrin und einem pharmazeutischen Salz davon, zur Verwendung bei der Behandlung von obstruktiver Kardiopathie.

2. Stoff zur Verwendung nach Anspruch 1, wobei das pharmazeutische Salz Hydrochlorid ist

3. Stoff zur Verwendung nach einem der Ansprüche 1 oder 2, wobei die obstruktive Kardiopathie ausgewählt ist aus hypertrophischer Kardiomyopathie, hypertensiver Kardiopathie, einer Fehlstellung von Papillarmuskeln und einer Situation mit primärer Hyperkinesie des linken Ventrikels bei nicht hypertrophischer Kardiomyopathie.

4. Stoff zur Verwendung nach einem der vorhergehenden Ansprüche, wobei die Behandlung eine Verabreichung einer Dosis des Stoffs umfasst, die 6 mg bis 45 mg pro Tag umfasst.

5. Stoff zur Verwendung nach einem der vorhergehenden Ansprüche, wobei die Behandlung eine Verabreichung einer Dosis des Stoffs umfasst, die von ein bis acht Mal pro 24 Stunden ausgeführt wird.

6. Stoff zur Verwendung nach einem der vorhergehenden Ansprüche, wobei die Behandlung die Verbesserung mindestens eines Symptoms der obstruktiven Kardiopathie einschließt, die ausgewählt ist aus Training von Atemlosigkeit, Training von Brustschmerzen, Training von Unwohlsein und Training von Schwindel.

7. Stoff zur Verwendung nach einem der vorhergehenden Ansprüche, wobei die Behandlung eine orale Verabreichung des Stoffs umfasst.

8. Stoff zur Verwendung nach Anspruch 7, wobei die orale Verabreichung eine bukkale oder sublinguale Verabreichung ist.

9. Stoff zur Verwendung nach Anspruch 7, wobei der Stoff in einer Form vorliegt, die ausgewählt ist aus der Gruppe umfassend eine flüssige Formulierung, eine orale Brausedosierungsform, ein orales Pulver, eine Pille, ein multipartikuläres System, eine orodispergierbare Dosierungsform, eine Lösung, ein Sirup, eine Suspension, eine Emulsion und orale Tropfen.

10. Stoff zur Verwendung nach einem der Ansprüche 1 bis 6, wobei die Behandlung eine parenterale Verabreichung des Stoffs umfasst.

11. Stoff zur Verwendung nach Anspruch 10, wobei die parenterale Verabreichung ausgewählt ist aus der Gruppe umfassend die intravenöse Verabreichung, intramuskuläre Verabreichung und subkutane Verabreichung.

12. Stoff zur Verwendung nach Anspruch 11, wobei der Stoff in Form einer injizierbaren Lösung vorliegt.

13. Stoff zur Verwendung nach einem der vorhergehenden Ansprüche für eine Verabreichung einem Menschen oder einem Tier, ausgewählt aus der Gruppe umfassend Feliden, Caniden, Cerviden, Equiden, Musteliden, Procyoniden, Viverriden und Ursiden.

14. Stoff zur Verwendung nach einem der vorhergehenden Ansprüche, zusammen mit mindestens einer weiteren aktiven Verbindung, die ausgewählt ist aus der Gruppe umfassend Betablocker, Calciumkanalblocker, Antiarrhythmika und Antikoagulanzien.

## Revendications

1. Substance sélectionnée parmi la midodrine, la desglymidodrine et un sel pharmaceutique de ceux-ci, pour son utilisation dans le traitement de la cardiopathie obstructive.

2. Substance pour utilisation selon la revendication 1, dans laquelle ledit sel pharmaceutique est le chlorhydrate.

3. Substance pour utilisation selon l'une quelconque des revendications 1 ou 2, dans laquelle la cardiopathie obstructive est sélectionnée parmi la cardiomyopathie hypertrophique, la cardiomyopathie hypertensive, malposition des muscles papillaires, et une situation avec hyperkinésie primaire du ventricule gauche dans une cardiomyopathie non hypertrophique.

4. Substance pour utilisation selon l'une quelconque des revendications précédentes, dans laquelle ledit traitement comprend une administration d'une dose de ladite substance de 6 mg à 45 mg par jour.

5. Substance pour utilisation selon l'une quelconque des revendications précédentes, dans laquelle ledit traitement comprend une administration de ladite substance qui est réalisée de une à huit fois par 24 heures.

6. Substance pour utilisation selon l'une quelconque des revendications précédentes, dans laquelle ledit traitement inclut l'amélioration d'au moins un symptôme de la cardiopathie obstructive sélectionnée parmi l'essoufflement à l'exercice, la douleur de poitrine à l'exercice, l'inconfort à l'exercice et les vertiges à l'exercice.

7. Substance pour utilisation selon l'une quelconque des revendications précédentes, dans laquelle ledit traitement comprend une administration orale de ladite substance.

8. Substance pour utilisation selon la revendication 7, dans laquelle ladite administration orale est une administration orale ou sublinguale.

9. Substance pour utilisation selon la revendication 7, dans laquelle ladite substance est dans une forme sélectionnée dans le groupe comprenant une formulation liquide, une forme orale effervescente, une poudre pour administration orale, une pilule, un système multiparticulaire, une forme orodispersible, une solution, un sirop, une suspension, une émulsion et des goutes pour administration orale.

10. Substance pour utilisation selon l'une quelconque des revendications 1 à 6, dans laquelle ledit traitement comprend une administration parentérale de ladite substance.

11. Substance pour utilisation selon la revendication 10, dans laquelle ladite administration parentérale est sélectionnée dans le groupe comprenant une administration intraveineuse, une administration intramusculaire et une administration subcutanée.

12. Substance pour utilisation selon la revendication 11, dans laquelle la substance est sous une forme d'une solution injectable.

13. Substance pour utilisation selon l'une quelconque des revendications précédentes, pour une administration à un être humain ou à un animal sélectionné dans le groupe comprenant les félidés, les canidés, les cervidés, les équidés, les mustélidés, les procyonidés, les viverridés et les ursidés.

14. Substance pour utilisation selon l'une quelconque des revendications précédentes, avec au moins une autre substance active sélectionnée dans le groupe comprenant les béta-bloquants, les bloqueurs des canaux calciques, les agents antiarrythmiques et les agents anticoagulants.
